# EUROPÄISCHE PATENTSCHRIFT

(11) **EP 4 039 235 B1**
(45) Veröffentlichungstag und Bekanntmachung des Hinweises auf die Patenterteilung: **14.10.2026**
(21) Anmeldenummer: 21155900.0
(22) Anmeldetag: 09.02.2021
(51) Int. Cl.: A61F 9/00, A61M 11/00, B65D 47/18, B65D 77/04

(54) **FLÜSSIGKEITSSPENDER MIT SICHERUNG**
FLUID DISPENSER WITH SECURING MEANS
DISTRIBUTEUR DE LIQUIDE SÉCURISÉ

(43) Veröffentlichungstag der Anmeldung: 10.08.2022
(60) Teilanmeldung: 26200989.7 / 4 818 073
(73) Patentinhaber: Aptar Radolfzell GmbH, 78315 Radolfzell (DE)
(72) Erfinder: Heinzle, Volker, 72505 Krauchenwies (DE); Trinkner, Julian, 78269 Volkertshausen DE (DE)
(74) Vertreter: Witte, Weller und Partner Patentanwälte mbB Stuttgart

(56) Entgegenhaltungen:
- EP-B1- 2 621 419
- DE-B3- 102016 210 992
- DE-U1- 202018 103 271

## Beschreibung

Die Erfindung betrifft einen Flüssigkeitsspender zur Abgabe pharmazeutischer Flüssigkeiten. Insbesondere betrifft die Erfindung Flüssigkeitsspender, die zur Abgabe von Flüssigkeiten in Form von Einzeltropfen vorgesehen sind, beispielsweise zur Applikation als Augentropfen in die Augen des Patienten.

Ein gattungsgemäßer Flüssigkeitsspender weist vorzugsweise einen Quetschflaschenkörper auf, der den Flüssigkeitsspeicher bildet. Die darin enthaltene Flüssigkeit kann durch beidseitige Kraftbeaufschlagung ausgetragen werden kann. Hierfür ist an einem Behälterhals des Quetschflaschenkörpers ein Austragkopf befestigt, der über eine Austragöffnung verfügt.

Die von der Erfindung betroffenen Flüssigkeitsspender beinhalten üblicherweise pharmazeutische Flüssigkeiten, deren Einnahme durch Kinder gesundheitlich nachteilig sein könnte. Dies ist insbesondere bei Quetschflaschenspendern problematisch, da ein Austrag hier selbst durch Kleinkinder zu bewerkstelligen ist, die ungezielt den Quetschflaschenkörper zusammendrücken und dadurch einen Flüssigkeitsaustrag bewirken.

Aus der DE 20 2018 103 271 U1 ist ein Flüssigkeitsspender mit einer kindergesicherten Kappe bekannt. Das Dokument schlägt eine Kappe mit Sicherungsdurchbrechungen vor, in die bei aufgesetzter Kappe eindrückbare Sicherungselemente einrücken.

EP 2 621 419 B1 offenbart einen Tropfenspender mit einem elastisch verformbaren Medienbehälter und einer abnehmbaren Kappe, die einen mantelförmigen Fortsatz mit Aussparungen aufweist, wobei der Fortsatz bei aufgesetzter Kappe den Medienbehälter zumindest abschnittsweise umgibt und ein unbeabsichtigtes Zusammendrücken des Medienbehälters verhindert oder erschwert.

### AUFGABE UND LÖSUNG

Aufgabe der Erfindung ist es, einen Flüssigkeitsspender dahingehend weiterzubilden, dass die Gefahr eines ungewollten Flüssigkeitsaustrags vermindert wird.

Gemäß der Erfindung wird ein Flüssigkeitsspender vorgeschlagen, der einen Quetschflaschenkörper mit einem Behälterhals aufweist, wobei am Behälterhals ein Austragkopf befestigt ist, der über eine Austragöffnung verfügt, durch die hindurch Flüssigkeit aus dem Quetschflaschenkörper austragbar ist. Vorzugsweise handelt es sich bei dem Spender um einen Tropfenspender zur Abgabe von Einzeltropfen, insbesondere zur ophthalmischen Anwendung. Der Austragkopf eines solchen Tropfenspenders verfügt vorzugsweise über Tropfenbildungsmittel, insbesondere in Form einer planen oder konischen Tropfenbildungsfläche, an der sich ausgetragene Flüssigkeit sammelt, bis sie sich als Tropfen von der Tropfenbildungsfläche löst. Ein solcher Tropfenspender weist vorzugsweise ein der Austragöffnung vorgeschaltetes Auslassventil auf, insbesondere wenn die enthaltene Flüssigkeit konservierungsmittelfrei ist.

Um das ungewollte Zusammendrücken des Quetschflaschenkörpers zu verhindern, insbesondere wenn Kleinkinder dies versuchen, weist der Flüssigkeitsspender gemäß dem ersten Aspekt der Erfindung einen ersten Blendenkörper auf, der seinerseits mindestens eine Sperrwandung aufweist und mindestens eine Betätigungsaussparung definiert. Dieser erste Blendenkörper ist unverlierbar und zwischen einer Sperrstellung und einer Freigabestellung beweglich am Flüssigkeitsspender vorgesehen und hierdurch gegenüber dem Quetschflaschenkörper verlagerbar.

In der Sperrstellung überdeckt der erste Blendenkörper mittels der Sperrwandung einen Teilbereich des Quetschflaschenkörpers, so dass ein Zusammendrücken des Quetschflaschenkörpers verhindert oder zumindest erschwert ist. In der Freigabestellung gibt der erste Blendenkörper mittels der Betätigungsaussparung den genannten Teilbereich des Quetschflaschenkörpers frei, so dass ein Zusammendrücken des Quetschflaschenkörpers ermöglicht ist.

Um den Flüssigkeitsspender zu verwenden, verlagert der Nutzer den Blendenkörper in die Freigabestellung. Nach Abschluss des Austragvorgangs verlagert er den Blendenkörper zurück in die Sperrstellung, in der ein ungewollter Flüssigkeitsaustrag unterbunden ist.

Der Quetschflaschenkörper kann durch seine Formgebung oder Wandungsgestaltung eine umfänglich uneinheitliche Eindrückbarkeit aufweisen. In einem solchen Fall ist der mindestens eine durch die Sperrwandung überdeckbare Teilbereich ein Flächenabschnitt, der ein besonders leichtes Eindrücken gestattet. Wenn sich der Blendenkörper in seiner Sperrstellung befindet, ist das Zusammendrücken des Quetschflaschenkörpers dadurch erschwert, dass nur ein schwerer oder ein nicht eindrückbarer Teilbereich des Quetschflaschenkörpers zugänglich ist.

Vorzugsweise ist zusätzlich ein zweiter Blendenkörper vorgesehen. Dieser weist ebenfalls mindestens eine Sperrwandung auf und definiert mindestens eine Betätigungsaussparung. Der erste Blenden körper ist gegenüber diesem zweiten Blendenkörper verlagerbar, so dass in der Freigabestellung die Betätigungsaussparungen derart überlappen oder sogar deckungsgleich sind, dass durch beide Betätigungsaussparungen hindurch eine Kraftbeaufschlagung des Quetschflaschenkörpers ermöglicht ist. Ist der ersten Blendenkörper dagegen in der Sperrstellung, so sind die Betätigungsaussparungen derart gegeneinander versetzt, dass eine Betätigung des Quetschflaschenkörpers verhindert oder zumindest erschwert ist.

Die Sperrabschnitte beider Blendenkörper können in der Sperrstellung insbesondere mittels ihrer Sperrabschnitte eine Mantelwandung des Quetschflaschenkörpers umfänglich vollständig oder nahezu vollständig (>80%) umgeben.

Die Blendenkörper sind vorzugsweise als den Quetschflaschenkörper umgebende Schalenkörper ausgebildet.

Der erste Blendenkörper ist vorzugsweise drehbar gegenüber dem Quetschflaschenkörper ausgebildet, insbesondere um eine Hauptachse, in der auch die Austragöffnung liegt, und/oder um eine Mittelachse des Quetschflaschenkörpers. Der zweite Blendenkörper ist vorzugsweise drehfest zum Quetschflaschenkörper an diesem angebracht, insbesondere vorzugsweise mittels einer Klemmverbindung. Es ist jedoch auch eine Gestaltung von der Erfindung umfasst, bei der beide Blendenkörper gegenüber dem Quetschflaschenkörper und gegeneinander beweglich und insbesondere drehbar sind.

Der erste und/oder der zweite Blendenkörper weisen vorzugsweise einen im wesentlichen rotationssymmetrischen Hülsenabschnitt auf, in dem die mindestens eine Betätigungsaussparung in Form einer Durchbrechung vorgesehen ist. Die Hülsenabschnitte weisen vorzugsweise eine Länge auf, die es ihnen gestattet, vom oberen Ende des Quetschflaschenkörpers bis zu dessen Boden zu ragen. Die mindestens eine Durchbrechung ist als allseitig vom jeweiligen Hülsenabschnitt umgebene Aussparung ausgebildet.

Eine mögliche Gestaltung sieht vor, dass genau eine Durchbrechung am ersten bzw. am zweiten Blendenkörper vorgesehen ist. Zum Zusammendrücken übt der Nutzer mit mindestens einem Finger durch die Durchbrechung hindurch unmittelbar eine Kraft auf den Quetschflaschenkörper aus. Gegenüberliegend zu der einen Durchbrechung ist keine weitere Durchbrechung vorgesehen, so dass hier unmittelbar auf den Hülsenabschnitt und nur mittelbar auf den Quetschflaschenkörper durch den Nutzer eine Kraft ausgeübt wird. Bei einer solchen Gestaltung mit nur einer Durchbrechung am ersten und gegebenenfalls am zweiten Blendenkörper überspannt diese Durchbrechung in Umfangsrichtung vorzugsweise einen Winkelbereich zwischen 120° bis 240°, insbesondere vorzugsweise einen Winkelbereich zwischen 150° und 210°.

Bevorzugt ist eine Gestaltung, bei der einander gegenüberliegend zwei Durchbrechungen am ersten bzw. am zweiten Blendenkörper vorgesehen sind. Zum Zusammendrücken des Quetschflaschenkörpers übt der Nutzer mit jeweils mindestens einem Finger, insbesondere mit Zeigefinger und Daumen, durch die einander gegenüberliegenden Durchbrechung hindurch beidseitig ein Kraft auf den Quetschflaschenkörper aus. Bei einer solchen Gestaltung ist vorzugsweise vorgesehen, dass die beiden Durchbrechungen jeweils einen Winkelbereich zwischen 60° und 170° überspannen, vorzugsweise jeweils einen Winkelbereich zwischen 90° und 160°.

Finden zwei Blendenkörper Verwendung, so sind diese vorzugsweise in Art einer Innenschale und einer Außenschale den Quetschflaschenspeicher umgebend angeordnet, wobei eine einfache Gestaltung vorsieht, dass der erste Blendenkörper außenliegend angeordnet ist und der zweite Blendenkörper innenliegend angeordnet und vorzugsweise drehfest am Quetschflaschenkörper vorgesehen ist. Die Sperrwandung des zweiten Blendenkörpers ist dadurch im gesperrten Zustand zwischen dem Quetschflaschenkörper und der Sperrwandung des ersten Blendenkörpers angeordnet.

Es ist jedoch auch eine Bauform möglich, bei der der zweite vorzugsweise ortsfest zum Quetschflaschenkörper vorgesehene Blendenkörper die Außenschale bildet und der erste bewegliche und vorzugsweise drehbare Blendenkörper die Innenschale bildet.

Der Quetschflaschenkörper, der erste Blendenkörper und gegebenenfalls der zweite Blendenkörper sind vorzugsweise als jeweils einstückige Bauteile aus Kunststoff gefertigt. Zur Montage wird vorzugsweise der erste und gegebenenfalls der zweite Blendenkörper von oben oder vorzugsweise von unten auf den Quetschflaschen körper geschoben. Vorzugsweise weist der erste und/oder der zweite Blendenkörper Rastmittel auf, um axial am anderen Blendenkörper oder am Quetschflaschenspeicher oder dem Austragkopf zu verrasten.

Die Blendenkörper können mindestens einen auslenkbaren Sicherungsabschnitt aufweisen, der beim Aufschieben des Blendenkörpers elastisch aufgeweitet wird und in der Endlage wieder zumindest teilweise entspannt. Insbesondere kann ein solcher elastisch auslenkbarer Sicherungsabschnitt am zweiten Blendenkörper vorgesehen sein, um nach Aufschieben des zweiten Blendenkörpers auf den Quetschflaschenkörper ein Herausziehen des Quetschflaschenkörpers aus dem zweiten Blendenkörper zu verhindern.

Insbesondere vorzugsweise weist der erste Blendenkörper eine Stützfläche auf, durch die nach Aufschieben des ersten Blendenkörpers auf den zweiten Blendenkörper im Zuge der Montage die elastische Auslenkbarkeit des Sicherungsabschnitts des zweiten Blendenkörpers beschränkt ist. Der erste Blendenkörper sichert demzufolge den zweiten Blendenkörper am Quetschflaschenkörper.

Es kann insbesondere auch eine Mehrzahl von radial auslenkbaren Sicherungsabschnitten vorgesehen sein. Vorzugsweise weist mindestens einer der Blendenkörper zwei solche Sicherungsabschnitte auf, wobei der Quetschflaschenkörper bei der Montage diese Sicherungsabschnitte voneinander weg drückt, um hindurch zu passen. Bei einer Gestaltung mit mehreren Sicherungsabschnitten ist vorzugsweise vorgesehen, dass eine gemeinsame und vorzugsweise umlaufende Stützfläche vorgesehen ist, deren radiale Auslenkbarkeit nach Abschluss der Montage beschränkt ist.

Es ist von Vorteil, wenn der erste Blendenkörper in mindestens einer der Stellungen, also der Sperrstellung und der Freigabestellung, verriegelbar ist. Zwar ist auch eine Gestaltung denkbar, bei der durch einen hohen Drehwiderstand die jeweilige Stellung lediglich reibschlüssig gehalten wird. Vorzugsweise ist jedoch eine schaltbare Verriegelung vorgesehen, mittels derer der erste Blendenkörper rotativ am Quetschflaschenkörper, am zweiten Blendenkörper oder am Austragkopf verriegelt werden kann. Diese Verriegelung erfolgt vorzugsweise durch formschlüssig wirkende Verriegelungselemente, die insbesondere einer Drehbewegung entgegenwirken können. Befinden sind die Verriegelungselemente im Eingriff miteinander, so ist eine Bewegung des ersten Blendenkörpers nicht möglich. Die Verriegelungselemente sind derart angeordnet, dass ein solcher verriegelter Zustand in der Sperrstellung oder in der Freigabestellung oder in beiden Stellungen möglich ist.

Um die Verriegelungselemente außer Eingriff zu bringen, wird vorzugsweise einer der Körper, insbesondere der erste Blendenkörper, elastisch verformt. Insbesondere kann hierfür vorgesehen sein, dass einer der Blendenkörper ein elastisch verformbares umlaufendes Ringsegment aufweist, an welchem einander gegenüber außenliegende Entriegelungsflächen vorgesehen sind und an welchem innenliegend ein in Umfangsrichtung zwischen den Entriegelungsflächen angeordnetes Verriegelungselement vorgesehen ist. Durch aufeinander zu gerichtete Kraftbeaufschlagung der Entriegelungsflächen wird das Ringsegment zusammengedrückt und das Verriegelungselement nach außen ausgelenkt, wodurch der Eingriff mit einem innenliegenden Verriegelungselement, insbesondere am zweiten Blendenkörper, gelöst wird.

Auch kann vorgesehen sein, dass am ersten Blendenkörper oder am zweiten Blendenkörper ein isoliert elastisch auslenkbares Verriegelungselement vorgesehen ist, welches durch manuelle Kraftbeaufschlagung verlagerbar ist, insbesondere verschwenkbar. Diese Verlagerung gestattet es dem auslenkbaren Verriegelungselement außer Eingriff von einem korrespondierenden Verriegelungselement am anderen Blendenkörper oder am Quetschflaschenkörper zu gelangen.

Da es insbesondere bevorzugt ist, dass die Blendenkörper zur Erzielung des Sperrzustandes bzw. des Freigabezustandes gegeneinander verdreht werden, kann es von Vorteil sein, wenn mindestens an einem der Blendenkörper hierfür eine Grifffläche vorgesehen ist. Insbesondere vorteilhaft ist eine Gestaltung, bei der der zweite Blendenkörper eine zumindest annähernd umlaufende Grifffläche aufweist. Diese ist vorzugsweise mit einer Strukturierung versehen, insbesondere mit einem regelmäßigen Muster von Erhöhungen oder Vertiefungen. Die Grifffläche ist vorzugsweise außenseitig an den oben beschriebenen auslenkbaren Sicherungsabschnitten vorgesehen.

Wie bereits dargestellt, ist der Flüssigkeitsspender gemäß der Erfindung vorzugsweise als Tropfenspender zur Abgabe von Einzeltropfen ausgebildet, insbesondere als Tropfenspender zur ophthalmischen Anwendung. In der Ausgestaltung als Tropfenspender weist der Flüssigkeitsspender vorzugsweise Tropfenbildungsmittel auf, insbesondere in Form einer planen oder konischen Tropfenbildungsfläche.

Der Flüssigkeitsspeicher eines erfindungsgemäßen Flüssigkeitsspenders, der insbesondere durch den beschriebenen Quetschflaschenkörper gebildet wird, weist vorzugsweise ein Aufnahmevolumen zwischen 2 ml und 100 ml auf, insbesondere vorzugsweise zwischen 4 ml und 15 ml.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert ist.
Fig. 1 zeigt ein erstes Ausführungsbeispiel eines erfindungsgemäßen Spenders.
Fig. 2A und 2B zeigen den Vorgang der Überführung des Spenders aus seinem Sperrzustand in seinen Freigabezustand.
Fig. 3A und 3B zeigen zwei Blendenkörper des Spenders.
Fig. 3C zeigt die Blendenkörper im montierten Zustand.
Fig. 4A bis 4C verdeutlichen die Montage des Spenders.

### DETAILLIERTE BESCHREIBUNG DES AUSFÜHRUNGSBEISPIELS

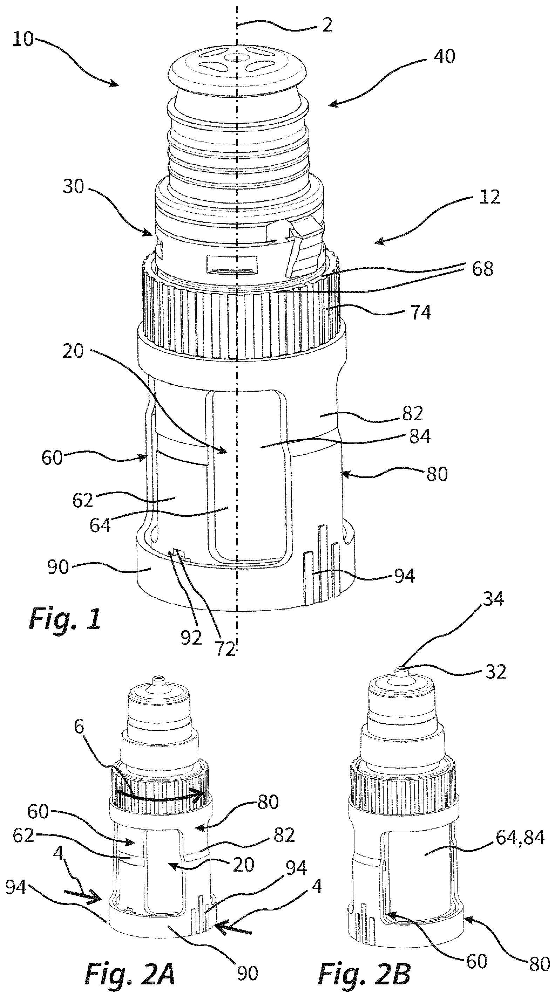

Fig. 1 zeigt einen Flüssigkeitsspender 10, der zum Austrag von Augentropfen dient, aber auch für andere pharmazeutische Anwendungen, insbesondere per Tropfenabgabe, ausgebildet sein kann.

Der Flüssigkeitsspender umfasst eine Austragvorrichtung 12 mit Flüssigkeitsspeicher in Form eines Quetschflaschenkörpers 20 und einen Austragkopf 30 sowie eine Kappe 40. Die Kappe 40 überdeckt eine Austragöffnung 32 sowie eine Tropfenbildungsfläche 34 am Austragkopf 30. Nach erstmaligem Abnehmen kann die Kappe 40 zum Zwecke der Nutzung des Flüssigkeitsspenders wiederholt abgenommen und aufgesetzt werden.

In nicht näher dargestellter Weise umfasst der Austragkopf 30 zusätzlich ein Auslassventil, welches durch Druckbeaufschlagung von Flüssigkeit im Quetschflaschenkörper 20 geöffnet werden kann, so dass Flüssigkeit zur Austragöffnung strömen kann und sich in Überkopflage der Austragvorrichtung 12 an der Tropfenbildungsfläche sammelt, bis sich der Tropfen löst.

Um eine einfache Handhabung und Dosierbarkeit zu gewährleisten, ist der Quetschflaschenkörper 20 bereits mit geringem Kraftaufwand zusammendrückbar. Dies geht jedoch mit der Gefahr einher, dass der Quetschflaschenkörper 20 ungewollt zusammengedrückt wird, beispielsweise in einer Tasche oder im Reisegepäck oder durch ein Kleinkind.

Um dies zu verhindern, ist eine Sicherung vorgesehen, die primär durch einen ersten Blendenkörper 80 und einen zweiten Blendenkörper 60 gebildet wird. Die beiden Blendenkörper 60, 80 umgeben im montierten Zustand der Fig. 1 den Quetschflaschenkörper 20. Beide Blendenkörper 60, 80 weisen eine grundsätzlich hülsenförmige oder in etwa zylindrische Form auf, wobei eine jeweilige Mantelfläche eine Sperrwandung 82, 62 bildet, die mit einander gegenüberliegenden Durchbrechungen 64, 84 versehen ist.

Mindestens einer der Blendenkörper 60, 80 ist gegenüber dem Quetschflaschenkörper 20 und gegenüber dem anderen Blendenkörper 60, 80 um die Mittelachse 2 drehbar ausgebildet, so dass je nach Relativdrehstellung der Blendenkörper 60, 80 zueinander die jeweiligen Durchbrechungen 64, 84 miteinander fluchten oder zueinander versetzt sind.

Fig. 2A zeigt den Zustand, in dem die Durchbrechungen 64, 84 zueinander versetzt sind und dadurch ein gesicherter Zustand der Austragvorrichtung 12 erzielt ist. Zwar schützen die Sperrwandungen 82, 62 bei der dargestellten Gestaltung aufgrund der Größe der Durchbrechungen 64, 84 den Quetschflaschenkörper 20 nicht umfänglich, die verbleibenden Öffnungen sind jedoch nicht groß genug, dass der Quetschflaschenkörper 20 ungewollt in relevantem Maße eingedrückt und hierdurch Flüssigkeit abgegeben werden kann.

Um ausgehend vom gesicherten Zustand der Fig. 2A einen Nutzzustand der Fig. 2B zu erzielen, müssen die Blendenkörper 60, 80 gegeneinander verdreht werden. Dies ist jedoch nicht allein mit einer einfachen Momentenbeaufschlagung möglich, da eine schaltbare Verriegelung 50 vorgesehen ist, die im verriegelten Zustand eine Drehbewegung formschlüssig unterbindet. Diese Verriegelung umfasst hierfür nach innen hervorragendes Verriegelungselemente 92 am ersten Blendenkörper 80, welche im verriegelten Zustand mit Verriegelungselementen 72 in Form von Wandungen von Vertiefungen am zweiten Blendenkörper 60 im Eingriff steht.

Um diese Verriegelung zu lösen muss ein den ersten Blendenkörper 80 unten abschließendes Ringsegment 90 verformt werden. Dieses weist hierfür einander gegenüberliegend Entriegelungsflächen 94 auf. Wird auf diese in Richtung der Pfeile 4 eine Kraft ausgeübt, so wird das Ringsegment 90 verformt und die Verriegelungselemente 92 werden radial nach außen verlagert, so dass der Eingriff mit der Verriegelungselementen 72 des zweiten Blendenkörpers 60 gelöst wird. In diesem entriegelten Zustand ist nun die Drehbewegung in Richtung des Pfeils 6 möglich, so dass der Nutzzustand der Fig. 2B erzielt ist.

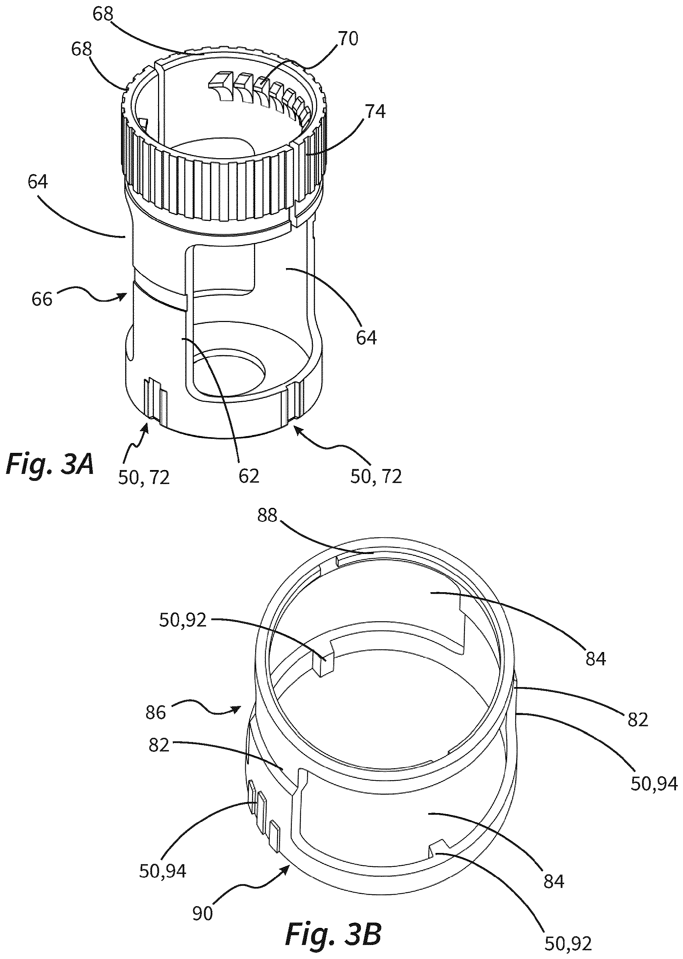

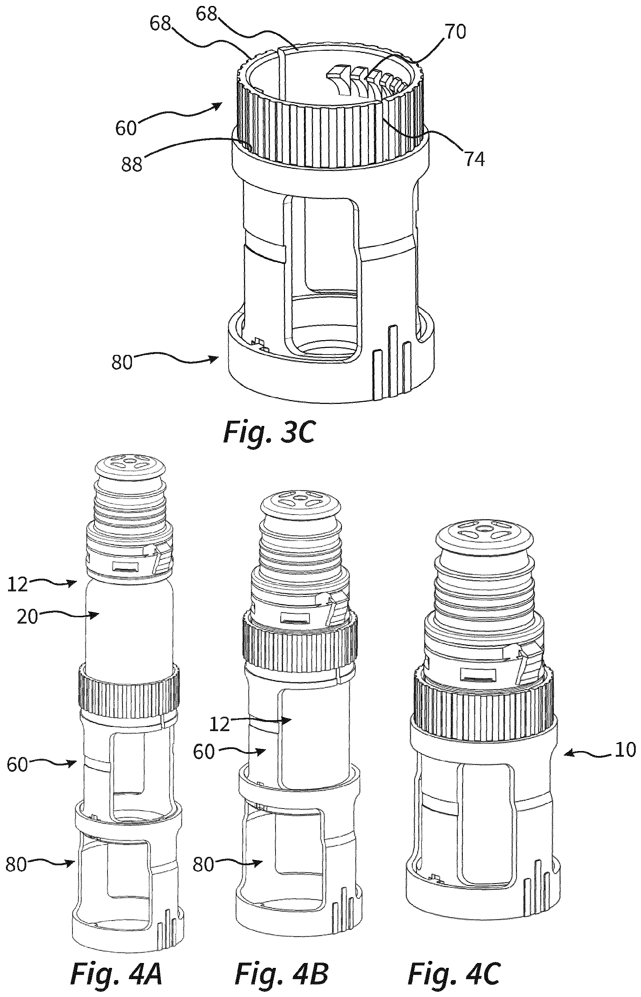

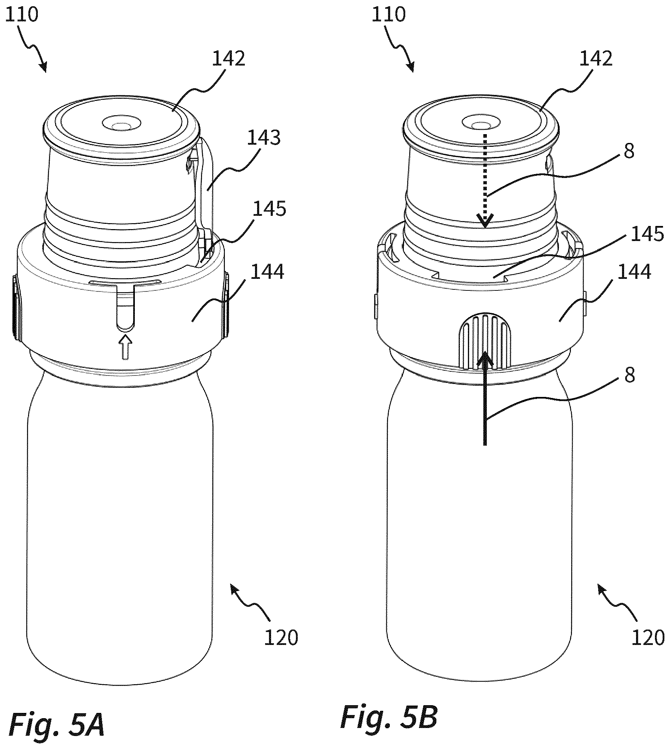

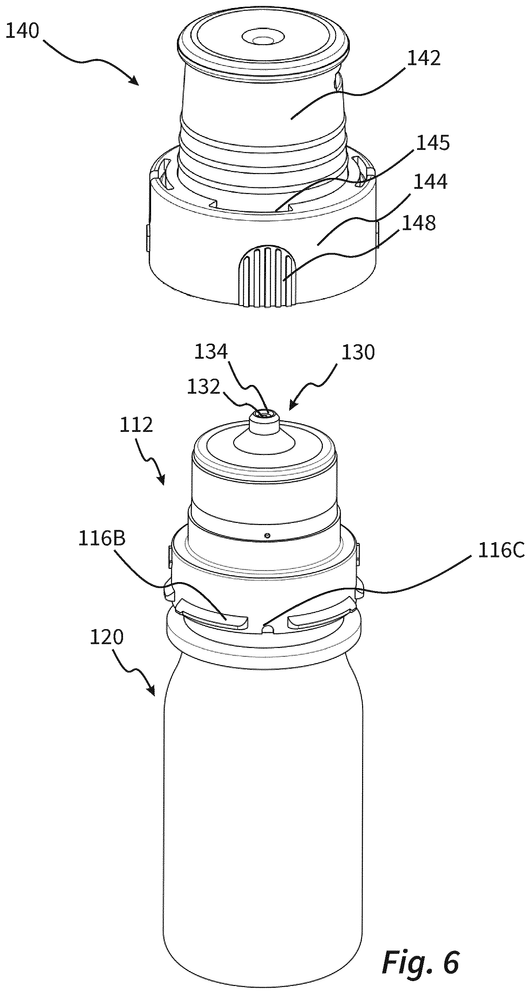

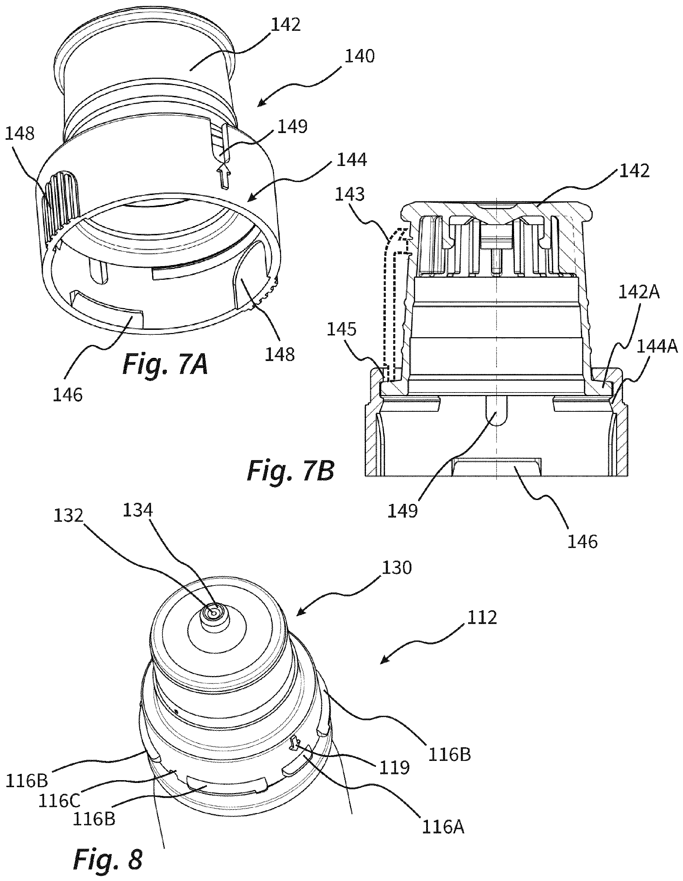

Die Fig. 3A bis 3C zeigen in jeweils separater sowie in montierter Darstellung die Blendenkörper 60, 80.

Fig. 3A zeigt den zweiten Blendenkörper 60, der beim vorliegenden Ausführungsbeispiel den inneren Blendenkörper bildet. Es ist zu erkennen, dass dessen Körper im Bereich des oberen Endes zweigeteilt ist. Zwei in etwa halbkreisförmige Sicherungsabschnitte 68 sind vorgesehen, deren Außenseite eine weitgehend umlaufende Grifffläche 74 bilden und die an der Innenseite eine Haltekontur 70 aufweisen. Durch die beschriebene Zweiteilung kann der zweite Blendenkörper 60 aufgeweitet werden, wenn er von unten auf den Quetschflaschenkörper 20 aufgeschoben wird. Weiterhin ist in Fig. 3A zu erkennen, dass insgesamt vier jeweils um 90° versetzte Vertiefungen vorgesehen sind, deren Flanken jeweils Verriegelungselemente 72 zum Zusammenwirken mit den Verriegelungselementen 92 des ersten Blendenkörpers bilden. Hierdurch ist eine Drehfixierung der Blendenkörper 60, 80 sowohl im gesicherten Zustand als auch im Nutzzustand möglich.

Bei dem in Fig. 3B dargestellten ersten Blendenkörper 80 ist in der Figur zu erkennen, dass ein oberer Ringabschnitt mit seiner Innenfläche eine Stützfläche bildet. Deren Innendurchmesser ist an den Außendurchmesser des zweiten Blendenkörpers unterhalb der Grifffläche 74 angepasst.

Dies führt dazu, dass im montierten Zustand der Blendenkörper 60, 80 die Stützfläche verhindert, dass die beiden halbkreisförmigen Sicherungsabschnitte 68 nach außen ausgelenkt werden. Hierdurch wird wiederum verhindert, dass sich der zweite Blendenkörper 60 nach Anbringung am Quetschflaschenkörper 20 von diesem lösen kann.

Fig. 4A bis 4C verdeutlichen die Montage. Wie Fig. 4A und 4B zeigen, wird zunächst die Austragvorrichtung 12, umfassend den Quetschflaschenkörper 20, in den zweiten Blendenkörper60 eingeschoben. Hierbei werden die genannten Sicherungsabschnitte 68 ausgelenkt. Sobald der Quetschflaschenkörper 20 in der in Fig. 4B dargestellten Art vollständig im zweiten Blendenkörper aufgenommen ist, sind die Sicherungsabschnitte 68 wieder in ihre Ausgangslage zurückgekehrt und halten den Quetschflaschenkörper mittels der Haltekontur 70.

Im nächsten Schritt wird nun in der aus Fig. 4B und 4C ersichtlichen Weise der erste Blendenkörper 80 aufgeschoben. Seine Stützfläche 88 verrastet unterhalb der Grifffläche 74 des zweiten Blendenkörpers 60, so dass eine Aufweitung des zweiten Blendenkörpers 60 nicht mehr möglich ist. Gleichzeitig ist der erste Blendenkörper 80 hierdurch auch gegen axiales Abziehen gesichert.

Die Fig. 4A bis 4C zeigen eine Gestaltung, bei der die beiden Blendenkörper 60, 80 bereits beim Ausgangszustand der Montage in Fig. 4A miteinander gekoppelt sich und eine Vormontageeinheit bilden. Wenngleich dies bevorzugt wird, ist es auch möglich, die Blendenkörper 60, 80 erst während der Montage miteinander zu verbinden.

## Patentansprüche

1. Flüssigkeitsspender (10) zur Abgabe pharmazeutischer Flüssigkeiten mit den folgenden Merkmalen:
a. der Flüssigkeitsspender (10) weist einen Quetschflaschenkörper (20) mit einem Behälterhals (22) auf, und
b. der Flüssigkeitsspender (10) weist einen am Quetschflaschenkörper (20) befestigten Austragkopf (30) auf, der über eine Austragöffnung (32) verfügt, durch die hindurch Flüssigkeit aus dem Quetschflaschenkörper (20) austragbar ist, und
c. der Flüssigkeitsspender (10) weist einen ersten Blendenkörper (80) auf, der mindestens eine Sperrwandung (82) aufweist und mindestens eine Betätigungsaussparung (84) definiert, und
d. der erste Blendenkörper (80) ist unverlierbar und beweglich am Flüssigkeitsspender vorgesehen, und
e. der erste Blendenkörper (80) überdeckt in einer Sperrstellung mittels der Sperrwandung (82) einen Teilbereich des Quetschflaschenkörpers (20), so dass ein Zusammendrücken des Quetschflaschenkörpers (20) verhindert oder zumindest erschwert ist, und gibt in einer Freigabestellung mittels der Betätigungsaussparung (84) den Teilbereich des Quetschflaschenkörpers (20) frei, so dass ein Zusammendrücken des Quetschflaschenkörpers (20) ermöglicht ist.

2. Flüssigkeitsspender nach Anspruch 1 mit den folgenden weiteren Merkmalen:
a. es ist ein zweiter Blendenkörper (60) vorgesehen,
b. der zweite Blendenkörper (60) weist mindestens eine Sperrwandung (62) auf und definiert mindestens eine Betätigungsaussparung (64), und
c. der erste Blendenkörper (80) ist gegenüber dem zweiten Blendenkörper (60) verlagerbar, so dass in der Freigabestellung die Betätigungsaussparungen (64, 84) derart überlappen, dass durch beide Betätigungsaussparungen hindurch eine Kraftbeaufschlagung des Quetschflaschenkörpers (20) ermöglicht ist, und so dass in der Sperrstellung die Betätigungsaussparungen (64, 84) derart gegeneinander versetzt sind, dass eine Betätigung des Quetschflaschenkörpers (20) verhindert oder zumindest erschwert ist,
vorzugsweise mit dem folgenden zusätzlichen Merkmal:
d. der zweite Blendenkörper (60) weist eine zumindest annähernd umlaufende Grifffläche (74) auf, die vorzugsweise mit einer Strukturierung, insbesondere durch ein regelmäßiges Muster von Erhöhungen oder Vertiefungen gebildet ist.

3. Flüssigkeitsspender (10) nach einem der vorstehenden Ansprüche mit dem folgenden weiteren Merkmal:
a. der erste Blendenkörper (80) ist drehbar gegenüber dem Quetschflaschenkörper (20) und/oder gegenüber dem zweiten Blendenkörper (80), wobei die Drehachse (2) vorzugsweise mit einer Mittelachse (2) des Quetschflaschenkörpers (20) übereinstimmt,
vorzugsweise mit mindestens einem der folgenden zusätzlichen Merkmale:
b. der zweite Blendenkörper (60) ist gegenüber dem Quetschflaschenkörper (20) drehfest, insbesondere durch eine Klemmverbindung zwischen dem zweiten Blendenkörper (60) und dem Quetschflaschenkörper (20), oder
a. der zweiten Blendenkörper (60) ist drehbar gegenüber dem Quetschflaschenkörper (20) und gegenüber dem ersten Blendenkörper (80).

4. Flüssigkeitsspender (10) nach einem der vorstehenden Ansprüche mit dem folgenden weiteren Merkmal:
a. der erste und/oder der zweite Blendenkörper (60, 80) weist einen im wesentlichen rotationssymmetrischen Hülsenabschnitt (66, 86) auf, in dem die mindestens eine Betätigungsaussparung (64, 84) in Form einer Durchbrechung (64, 84) vorgesehen ist,
vorzugsweise mit mindestens einem der folgenden zusätzlichen Merkmale:
b. es ist genau eine Durchbrechung (64, 84) am ersten bzw. am zweiten Blendenkörper (60, 80) vorgesehen, wobei vorzugsweise diese Durchbrechung (64, 84) in Umfangsrichtung einen Winkelbereich zwischen 120° bis 240° überspannt, insbesondere vorzugsweise einen Winkelbereich zwischen 150° und 210°, oder
c. es sind zwei Durchbrechungen (64, 84) am ersten bzw. am zweiten Blendenkörper (60, 80) vorgesehen, die jeweils einen Winkelbereich zwischen 60° und 170° überspannen, vorzugsweise jeweils einen Winkelbereich zwischen 90° und 160°.

5. Flüssigkeitsspender (10) nach einem der Ansprüche 2 bis 4 mit dem folgenden weiteren Merkmal:
a. die beiden Blendenkörper (60, 80) sind derart relativ zueinander angeordnet, dass die Sperrwandung (62) des zweiten Blendenkörpers (60) im gesperrten Zustand zwischen dem Quetschflaschenkörper (20) und der Sperrwandung (82) des ersten Blendenkörpers (80) angeordnet ist.

6. Flüssigkeitsspender (10) nach einem der Ansprüche 2 bis 5 mit den folgenden weiteren Merkmalen:
a. der zweite Blendenkörper (60) weist mindestens einen elastisch auslenkbaren Sicherungsabschnitt (68) auf, der ein Herausziehen des Quetschflaschenkörpers (20) aus dem zweiten Blendenkörper (60) verhindert, und
b. der erste Blendenkörper (80) weist eine Stützfläche (88) auf, durch die die elastische Auslenkbarkeit des Sicherungsabschnitts (68) des zweiten Blendenkörpers (60) beschränkt ist,
insbesondere vorzugsweise mit mindestens einem der folgenden zusätzlichen Merkmale:
c. die Stützfläche (88) liegt außenseitig am zweiten Blendenkörper (60) im Bereich des elastisch auslenkbaren Sicherungsabschnitts (68) am zweiten Blendenkörper (60) an, und/oder
d. es sind eine Mehrzahl von radial auslenkbaren Sicherungsabschnitten (68) vorgesehen, wobei eine gemeinsame und vorzugsweise umlaufende Stützfläche (88) die radiale Auslenkbarkeit beschränkt.

7. Flüssigkeitsspender (10) nach einem der vorstehenden Ansprüche mit dem folgenden weiteren Merkmal:
a. es ist eine schaltbare Verriegelung (50) vorgesehen, mittels derer der erste Blendenkörper (80) in seiner Sperrstellung rotativ verriegelt werden kann,
vorzugsweise mit mindestens einem der folgenden zusätzlichen Merkmale:
b. der erste Blendenkörper (80) ist in der Sperrstellung durch formschlüssig wirkende Verriegelungselemente (92, 72) rotativ verriegelbar, und/oder
c. der erste Blendenkörper (80) ist mittels der Verriegelung am zweiten Blendenkörper (60) oder am Quetschflaschenkörper (20) rotativ gesichert, und/oder
d. mittels der schaltbaren Verriegelung (50) kann der erste Blendenkörper (80) auch in seiner Freigabestellung rotativ gesichert werden.

8. Flüssigkeitsspender (10) nach einem der vorstehenden Ansprüche mit dem folgenden weiteren Merkmal:
a. der erste Blendenkörper (80) weist ein elastisch verformbares umlaufendes Ringsegment (90) auf, an welchem einander gegenüber außenliegende Entriegelungsflächen (94) vorgesehen sind und an welchem innenliegend ein in Umfangsrichtung zwischen den Entriegelungsflächen (94) mindestens ein Verriegelungselement (92) vorgesehen ist.

9. Flüssigkeitsspender (10) nach einem der vorstehenden Ansprüche mit dem folgenden weiteren Merkmal:
a. am ersten Blendenkörper (80) oder am zweiten Blendenkörper (60) ist ein elastisch auslenkbares Verriegelungselement vorgesehen, welches durch manuelle Kraftbeaufschlagung verlagerbar ist, insbesondere verschwenkbar, so dass es von einem korrespondierenden Verriegelungselement am anderen Blendenkörper oder am Quetschflaschenkörper getrennt wird.

10. Flüssigkeitsspender (10) nach einem der vorstehenden Ansprüche mit dem folgenden weiteren Merkmal:
a. der Flüssigkeitsspender (10) ist als Tropfer zur Abgabe von Einzeltropfen ausgebildet, insbesondere als Tropfer zur ophthalmischen Anwendung,
vorzugsweise mit dem folgenden zusätzlichen Merkmal:
b. der Flüssigkeitsspender (10) verfügt über Tropfenbildungsmittel (34), insbesondere in Form einer planen oder konischen Tropfenbildungsfläche.

## Claims

1. Liquid dispenser (10) for dispensing pharmaceutical liquids, having the following features:
a. the liquid dispenser (10) has a squeezable bottle body (20) with a container neck (22), and
b. the liquid dispenser (10) has a delivery head (30), which is fastened to the squeezable bottle body (20) and has a discharge opening (32) through which liquid can be delivered from the squeezable bottle body (20), and
c. the liquid dispenser (10) has a first cover body (80), which has at least one blocking wall (82) and defines at least one actuating cutout (84), and
d. the first cover body (80) is provided so as to be captive and movable on the liquid dispenser, and
e. the first cover body (80), in a blocking position, covers a partial region of the squeezable bottle body (20) by means of the blocking wall (82), such that compression of the squeezable bottle body (20) is prevented or at least impeded, and, in an open position, opens the partial region of the squeezable bottle body (20) by means of the actuating cutout (84), such that compression of the squeezable bottle body (20) is made possible.

2. Liquid dispenser according to Claim 1, having the following further features:
a. a second cover body (60) is provided,
b. the second cover body (60) has at least one blocking wall (62) and defines at least one actuating cutout (64), and
c. the first cover body (80) can be displaced relative to the second cover body (60), such that, in the open position, the actuating cutouts (64, 84) overlap in such a manner that it is possible to apply force to the squeezable bottle body (20) through both actuating cutouts, and such that, in the blocking position, the actuating cutouts (64, 84) are offset relative to one another in such a manner that actuation of the squeezable bottle body (20) is prevented or at least impeded,
preferably having the following additional feature:
d. the second cover body (60) has an at least approximately circumferential gripping surface (74), which is preferably formed with structuring, in particular by a regular pattern of elevations or depressions.

3. Liquid dispenser (10) according to either of the preceding claims, having the following further feature:
a. the first cover body (80) can be rotated relative to the squeezable bottle body (20) and/or relative to the second cover body (80), wherein the axis of rotation (2) preferably coincides with a central axis (2) of the squeezable bottle body (20),
preferably having at least one of the following additional features:
b. the second cover body (60) is rotationally fixed relative to the squeezable bottle body (20), in particular by a clamped connection between the second cover body (60) and the squeezable bottle body (20), or
a. the second cover body (60) can be rotated relative to the squeezable bottle body (20) and relative to the first cover body (80).

4. Liquid dispenser (10) according to any of the preceding claims, having the following further feature:
a. the first and/or the second cover body (60, 80) has a substantially rotationally symmetrical sleeve portion (66, 86), in which the at least one actuating cutout (64, 84) is provided in the form of an aperture (64, 84),
preferably having at least one of the following additional features:
b. exactly one aperture (64, 84) is provided in the first or in the second cover body (60, 80), wherein preferably this aperture (64, 84) spans an angular range of between 120° and 240°, particularly preferably an angular range of between 150° and 210°, in the circumferential direction, or
c. two apertures (64, 84) are provided in the first or in the second cover body (60, 80), each of said apertures spanning an angular range of between 60° and 170°, preferably an angular range of between 90° and 160°.

5. Liquid dispenser (10) according to any of Claims 2 to 4, having the following further feature:
a. the two cover bodies (60, 80) are arranged relative to one another in such a manner that, in the blocked state, the blocking wall (62) of the second cover body (60) is arranged between the squeezable bottle body (20) and the blocking wall (82) of the first cover body (80).

6. Liquid dispenser (10) according to any of Claims 2 to 5, having the following further features:
a. the second cover body (60) has at least one elastically deflectable securing portion (68), which prevents the squeezable bottle body (20) from being pulled out of the second cover body (60), and
b. the first cover body (80) has a support surface (88), which is used to limit the elastic deflectability of the securing portion (68) of the second cover body (60),
particularly preferably having at least one of the following additional features:
c. the support surface (88) bears against the outside of the second cover body (60) in the region of the elastically deflectable securing portion (68) on the second cover body (60), and/or
d. a plurality of radially deflectable securing portions (68) are provided, wherein a common and preferably circumferential support surface (88) limits the radial deflectability.

7. Liquid dispenser (10) according to any of the preceding claims, having the following further feature:
a. a switchable locking means (50) is provided, which can be used to rotationally lock the first cover body (80) in its blocking position,
preferably having at least one of the following additional features:
b. the first cover body (80) can be rotationally locked in the blocking position by positive-locking elements (92, 72), and/or
c. the first cover body (80) is rotationally secured on the second cover body (60) or on the squeezable bottle body (20) by means of the locking means, and/or
d. the first cover body (80) can also be rotationally secured in its open position by means of the switchable locking means (50).

8. Liquid dispenser (10) according to any of the preceding claims, having the following further feature:
a. the first cover body (80) has an elastically deformable circumferential ring segment (90), on which mutually opposing external unlocking surfaces (94) are provided and on which at least one locking element (92) is provided internally in the circumferential direction between the unlocking surfaces (94).

9. Liquid dispenser (10) according to any of the preceding claims, having the following further feature:
a. on the first cover body (80) or on the second cover body (60), an elastically deflectable locking element is provided, which can be displaced, in particular pivoted, by manual application of force, such that it is separated from a corresponding locking element on the other cover body or on the squeezable bottle body.

10. Liquid dispenser (10) according to any of the preceding claims, having the following further feature:
a. the liquid dispenser (10) is designed as a dropper for delivering single drops, in particular as a dropper for ophthalmic use,
preferably having the following additional feature:
b. the liquid dispenser (10) has droplet forming means (34), in particular in the form of a planar or conical droplet forming surface.

## Revendications

1. Distributeur de liquide (10) pour la distribution de liquides pharmaceutiques, présentant les caractéristiques suivantes :
a. le distributeur de liquide (10) présente un corps de flacon compressible (20) muni d'un col de récipient (22), et
b. le distributeur de liquide (10) présente une tête de distribution (30) fixée au corps de flacon compressible (20), qui comporte une ouverture de distribution (32), à travers laquelle un liquide peut être distribué depuis le corps de flacon compressible (20), et
c. le distributeur de liquide (10) présente un premier corps de cache (80), qui présente au moins une paroi d'obturation (82) et définit au moins un évidement d'actionnement (84), et
d. le premier corps de cache (80) est prévu sur le distributeur de liquide de manière imperdable et mobile, et
e. le premier corps de cache (80), dans une position de blocage, recouvre au moyen de la paroi d'obturation (82) une zone partielle du corps de flacon compressible (20), de sorte qu'un compression du corps de flacon compressible (20) est empêché ou au moins rendu plus difficile, et, dans une position de libération, libère, au moyen de l'évidement d'actionnement (84), la zone partielle du corps de flacon compressible (20), de sorte qu'un compressioin du corps de flacon compressible (20) est rendu possible.

2. Distributeur de liquide selon la revendication 1, présentant les caractéristiques supplémentaires suivantes :
a. un deuxième corps de cache (60) est prévu,
b. le deuxième corps de cache (60) présente au moins une paroi d'obturation (62) et définit au moins un évidement d'actionnement (64), et
c. le premier corps de cache (80) est déplaçable par rapport au deuxième corps de cache (60), de sorte que, dans la position de libération, les évidements d'actionnement (64, 84) se chevauchent de telle manière qu'une application d'une force sur le corps de flacon compressible (20) à travers les deux évidements d'actionnement est rendue possible, et de sorte que, dans la position de blocage, les évidements d'actionnement (64, 84) sont décalés l'un par rapport à l'autre de telle manière qu'un actionnement du corps de flacon compressible (20) est empêché ou au moins rendu plus difficile,
de préférence avec la caractéristique supplémentaire suivante :
d. le deuxième corps de cache (60) présente une surface de préhension (74) s'étendant au moins approximativement sur tout le pourtour, qui est de préférence pourvue d'une structuration, notamment formée par un motif régulier de saillies ou de dépressions.

3. Distributeur de liquide (10) selon l'une des revendications précédentes, présentant la caractéristique supplémentaire suivante :
a. le premier corps de cache (80) est rotatif par rapport au corps de flacon compressible (20) et/ou par rapport au deuxième corps de cache (80), l'axe de rotation (2) coïncidant de préférence avec un axe central (2) du corps de flacon compressible (20),
de préférence avec au moins une des caractéristiques supplémentaires suivantes :
b. le deuxième corps de cache (60) est solidaire en rotation du corps de flacon compressible (20), notamment par une liaison par serrage entre le deuxième corps de cache (60) et le corps de flacon compressible (20), ou
a. le deuxième corps de cache (60) est rotatif par rapport au corps de flacon compressible (20) et par rapport au premier corps de cache (80).

4. Distributeur de liquide (10) selon l'une des revendications précédentes, présentant la caractéristique supplémentaire suivante :
a. le premier et/ou le deuxième corps de cache (60, 80) présente une section de manchon (66, 86) essentiellement à symétrie de rotation, dans laquelle l'au moins un évidement d'actionnement (64, 84) est prévu sous la forme d'une percée (64, 84),
de préférence avec au moins une des caractéristiques supplémentaires suivantes :
b. il est prévu exactement une percée (64, 84) sur le premier ou le deuxième corps de cache (60, 80), cette percée (64, 84) s'étendant de préférence, dans la direction circonférentielle, sur une plage angulaire comprise entre 120° et 240°, notamment de préférence sur une plage angulaire comprise entre 150° et 210°, ou
c. il est prévu deux percées (64, 84) sur le premier ou le deuxième corps de cache (60, 80), qui s'étendent chacune sur une plage angulaire comprise entre 60° et 170°, de préférence chacune sur une plage angulaire comprise entre 90° et 160°.

5. Distributeur de liquide (10) selon l'une des revendications 2 à 4 avec la caractéristique supplémentaire suivante :
a. les deux corps de cache (60, 80) sont disposés l'un par rapport à l'autre de telle manière que la paroi d'obturation (62) du deuxième corps de cache (60), dans l'état de blocage, est disposée entre le corps de flacon compressible (20) et la paroi d'obturation (82) du premier corps de cache (80).

6. Distributeur de liquide (10) selon l'une des revendications 2 à 5 avec les caractéristiques supplémentaires suivantes :
a. le deuxième corps de cache (60) présente au moins une section d'immobilisation (68) déviable de manière élastique, qui empêche un retrait du corps de flacon compressible (20) hors du deuxième corps de cache (60), et
b. le premier corps de cache (80) présente une surface d'appui (88), par laquelle la déviabilité élastique de la section d'immobilisation (68) du deuxième corps de cache (60) est limitée,
présentant notamment de préférence au moins l'une des caractéristiques supplémentaires suivantes :
c. la surface d'appui (88) est en appui du côté extérieur sur le deuxième corps de cache (60), dans la zone de la section d'immobilisation (68) déviable de manière élastique du deuxième corps de cache (60), et/ou
d. il est prévu une pluralité de sections d'immobilisation (68) déviables radialement, une surface d'appui (88) commune et de préférence périphérique limitant la déviabilité radiale.

7. Distributeur de liquide (10) selon l'une des revendications précédentes, présentant la caractéristique supplémentaire suivante :
a. un verrouillage commutable (50) est prévu, au moyen duquel le premier corps de cache (80) peut être verrouillé en rotation dans sa position de blocage,
de préférence avec au moins une des caractéristiques supplémentaires suivantes :
b. le premier corps de cache (80) peut être verrouillé en rotation dans la position de blocage par des éléments de verrouillage (92, 72) agissant par complémentarité de forme, et/ou
c. le premier corps de cache (80) est immobilisé en rotation au moyen du verrouillage sur le deuxième corps de cache (60) ou sur le corps de flacon compressible (20), et/ou
d. au moyen du verrouillage commutable (50), le premier corps de cache (80) peut également être immobilisé en rotation dans sa position de libération.

8. Distributeur de liquide (10) selon l'une des revendications précédentes, présentant la caractéristique supplémentaire suivante :
a. le premier corps de cache (80) présente un segment annulaire périphérique (90) élastiquement déformable, sur lequel sont prévues, à l'extérieur, des surfaces de déverrouillage (94) opposées l'une à l'autre et sur lequel est prévu, à l'intérieur, dans la direction circonférentielle entre les surfaces de déverrouillage (94), au moins un élément de verrouillage (92).

9. Distributeur de liquide (10) selon l'une des revendications précédentes, présentant la caractéristique supplémentaire suivante :
a. un élément de verrouillage déviable de manière élastique est prévu sur le premier corps de cache (80) ou sur le deuxième corps de cache (60), lequel est déplaçable par application manuelle d'une force, en particulier pivotable, de sorte qu'il est séparé d'un élément de verrouillage correspondant sur l'autre corps de cache ou sur le corps de flacon compressible.

10. Distributeur de liquide (10) selon l'une des revendications précédentes, présentant la caractéristique supplémentaire suivante :
a. le distributeur de liquide (10) est conçu sous la forme d'un compte-gouttes destiné à distribuer des gouttes individuelles, notamment d'un compte-gouttes destiné à une application ophtalmique,
de préférence avec la caractéristique supplémentaire suivante :
b. le distributeur de liquide (10) comporte des moyens de formation de gouttes (34), notamment sous la forme d'une surface de formation de gouttes plane ou conique.
